# FASCICULE DE BREVET EUROPEEN

(11) **EP 0 903 128 B1**
(45) Date de publication et mention de la délivrance du brevet: **19.05.2004**
(21) Numéro de dépôt: 98420153.3
(22) Date de dépôt: 10.09.1998
(51) Int. Cl.: A61F 2/40

(54) **Prothèse humérale à sphère indexée**
Humerusprothese mit indexierter Kugel
Humeral prosthesis with indexed sphere

(30) Priorité: 12.09.1997 FR 9711603
(43) Date de publication de la demande: 24.03.1999
(73) Titulaire: Tornier SA, 38330 Saint Ismier (FR)
(72) Inventeur: Tornier, Alain, 38330 Saint Ismier (FR)
(74) Mandataire: Schmitt, John

(56) Documents cités:
- EP-A- 0 549 480
- EP-A- 0 639 359
- EP-A- 0 679 375
- EP-A- 0 712 617
- EP-A- 0 715 836
- WO-A-96/17553
- WO-A-97/25943
- DE-C- 19 509 037

## Description

La présente invention a trait à des perfectionnements apportés aux prothèses d'épaule à sphère du genre comprenant une tige qui s'ancre dans le canal huméral et une calotte hémisphérique propre à coopérer avec la glène de l'épaule.

La prothèse suivant l'invention associée ou non à une glène prothétique permet le traitement chirurgical des affections dégénératives de l'arthrose gléno-humérale, mais aussi d'autres affections. En outre la prothèse suivant l'invention est destinée également aux fractures céphalo-tubérositaires non accessibles à une chirurgie conservatrice, mais encore à tout syndrome douloureux de l'épaule.

On connaît d'après la demande de brevet d'invention français N° 2 727 002 appartenant au demandeur une prothèse humérale à sphère comportant dans la partie métaphysaire de la tige humérale un logement cylindrique à fond sphérique dans lequel est introduite une sphère prévue pour recevoir une calotte hémisphérique, tandis que des moyens de serrage permettent leur immobilisation dans une position déterminée par rapport à l'axe de la tige.

On connaît également d'après la demande de brevet d'invention français N° 2 685 633 appartenant au demandeur une prothèse humérale modulaire, comportant trois éléments dont une tige d'ancrage pourvue d'une partie métaphysaire formée d'une face d'appui oblique. Le second élément est une entretoise en forme de coin dont l'une des faces extrêmes s'ajuste sur la face d'appui de la tige. Le troisième élément de la prothèse est une calotte hémisphérique dont la base se fixe par rapport à la deuxième face extrême de l'entretoise. Cette fixation, qui est excentrée par rapport à l'axe géométrique de la calotte, permet un réglage angulaire de cette dernière par rapport à l'entretoise.

De telles prothèses comportent certains inconvénients en ce qui conceme les systèmes de blocage des sphères qui sont inaccessibles dès que les prothèses sont introduites dans le canal huméral car les vis de serrage sont disposées à l'intérieur de la cavité osseuse.

De plus, on remarque qu'il existe, du fait de l'utilisation d'une sphère, une infinité de positions relatives entre la tige et la sphère, ce qui rend impossible la reproduction sur l'implant de la géométrie mesurée à l'aide de la pièce d'essai.

Les perfectionnements qui font l'objet de la présente invention visent à permettre la réalisation d'une prothèse humérale qui remédie aux inconvénients ci-dessus.

La prothèse humérale à sphère suivant la présente invention comprend une tige pouvant s'ancrer dans le canal huméral, une calotte hémisphérique propre à coopérer avec la glène de l'épaule et une sphère réalisant la liaison entre la tige et la calotte, et des moyens d'indexation qui sont constitués par la combinaison d'un index fixe avec un repère et une empreinte en face de l'axe d'un trou pour permettre l'indexation de la sphère et/ou la calotte par rapport à la tige dans un nombre fini de positions angulaires combinées α et β de la calotte par rapport à la tige qui sont obtenues des mesures relevées sur une pièce d'essai ou tout autre moyen afin de bloquer par des moyens de serrage ladite sphère et/ou ladite calotte sur la tige avant sa mise en place dans le canal huméral, lesdits angles α et β représentant respectivement l'angle d'inclinaison dans le plan frontal, et l'angle d'ante ou rétroversion par rapport au plan frontal autour de l'axe de la tige.

La prothèse humérale comprend un trou recevant les moyens de serrage qui est disposé suivant un axe non concourant avec le centre de la sphère et/ou la calotte pour empêcher tout mouvement relatif entre la sphère et/ou la calotte par rapport à la tige.

La prothèse humérale comprend une tige qui comporte dans sa partie métaphysaire une cavité propre à recevoir une sphère coopérant avec la calotte, tandis que des moyens de serrage prévus sur la tige permettent l'immobilisation de la sphère et de la calotte.

La prothèse humérale comprend une tige qui est solidaire dans sa partie métaphysaire d'une sphère qui coopère avec un logement ménagé dans la calotte, tandis que des moyens de serrage prévus sur la calotte permettent son immobilisation sur la sphère.

La prothèse humérale comprend une tige qui comporte dans sa partie métaphysaire un logement au milieu duquel est percé un trou à profil tronconique recevant un pion de même profil s'étendant d'une collerette disposée dans le logement, ladite collerette étant solidaire à l'opposé du pion d'une sphère qui coopère avec le logement prévu dans la calotte.

La prothèse humérale comprend un axe de la sphère qui est décalé latéralement d'une distance par rapport à l'axe de la collerette pour permettre une excentration de la calotte.

La description qui va suivre en regard des dessins annexés, donnés à titre d'exemples non limitatifs, permettra de mieux comprendre l'invention, les caractéristiques qu'elle présente et les avantages qu'elle est susceptible de procurer:
Figure 1 est une vue frontale représentant la prothèse humérale suivant la présente invention dans une première position angulaire a de la calotte par rapport à la tige de la prothèse.
Figure 2 est une vue de dessus montrant la prothèse humérale dans sa position angulaire de figure 1.
Figure 3 est vue frontale illustrant la prothèse humérale dans une seconde position angulaire α de la calotte, d'une valeur différente de celle prévue en figure 1, de la calotte par rapport à la tige de la prothèse.
Figure 4 est une vue de dessus montrant la prothèse humérale suivant la présente invention dans sa position angulaire de figure 2.
Figure 5 est une vue frontale représentant la prothèse humérale dans une troisième position angulaire.
Figure 6 est une vue de dessus illustrant la prothèse humérale dans sa position angulaire de figure 5, suivant un angle β par rapport au plan frontal de la tige de la prothèse.
Figure 7 est une vue montrant une première variante de la prothèse humérale suivant la présente invention.
Figure 8 est une vue représentant une seconde variante de la prothèse humérale suivant la présente invention.
Figure 9 est une vue illustrant une troisième variante de la prothèse humérale suivant la présente invention.

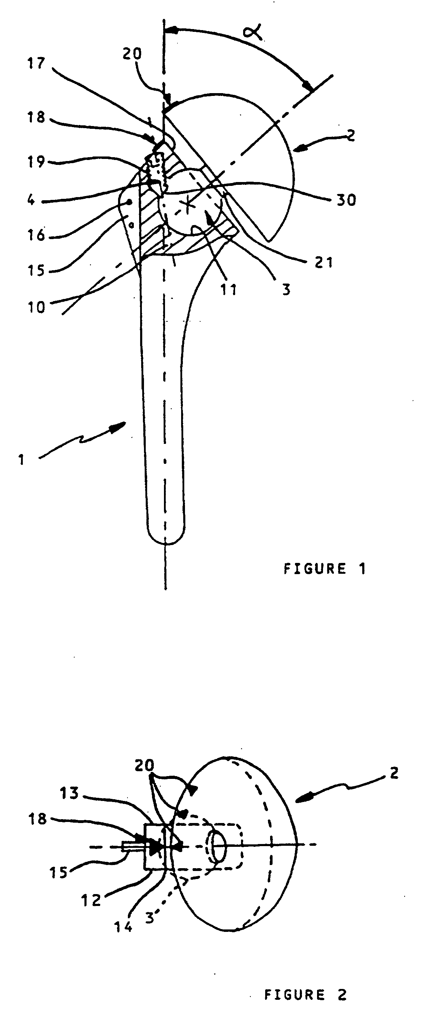

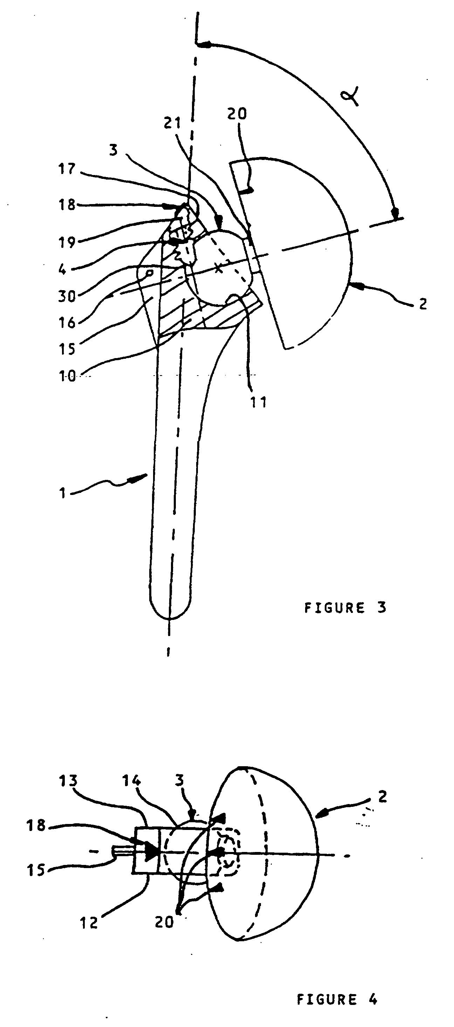

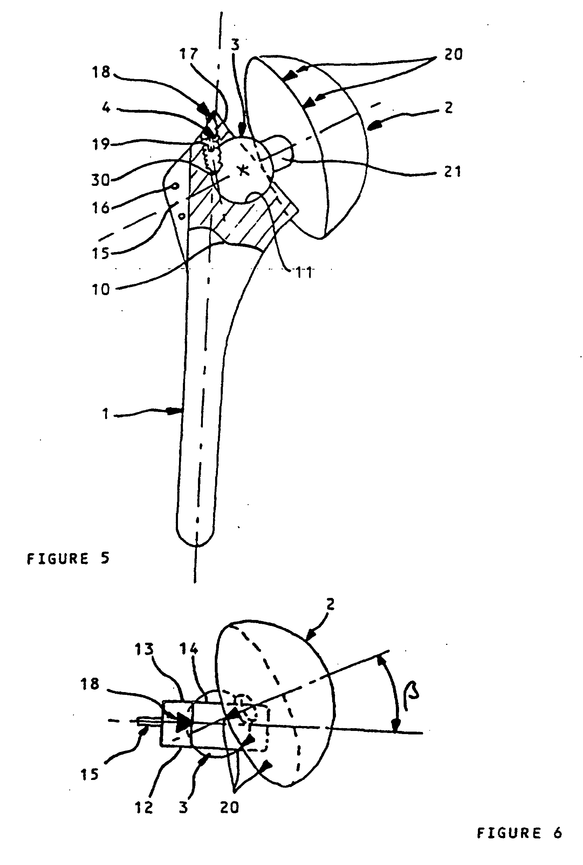

On a représenté en figures 1 à 6 les trois éléments de la prothèse humérale suivant l'invention, à savoir une tige 1 pouvant s'ancrer dans le canal huméral, une calotte hémisphérique 2 propre à coopérer avec la glène de l'épaule et une sphère 3 permettant la liaison mécanique entre la tige 1 et le calotte 2.

La tige 1 est de section cylindrique comportant une partie métaphysaire 10, à profil évasée, qui est pourvue d'une cavité 11 dans laquelle coopère la sphère 3. Dans le plan sagittal, la partie métaphysaire 10 comporte des flans droits et parallèles 12 et 13 définissant son épaisseur. On note que l'épaisseur de la partie métaphysaire 10 est inférieure au diamètre de la sphère 3. En effet les flans 12 et 13 peuvent être percés chacun d'un trou ou encoche 14 débouchant à l'intérieur de la cavité 11 et laissant dépasser à l'extérieur les pôles de la sphère 3.

La partie métaphysaire 10 comprend un ou plusieurs ailerons 15 qui sont disposés sur la face externe et/ou latérale de la tige 1, tandis que des perforations 16 sont ménagés dans les ailerons 15 pour permettre, en cas de fracture, une reconstitution de l'extrémité supérieure de l'humérus autour de la prothèse.

La tige 10 se termine par une face 17 qui est inclinée par rapport à l'axe longitudinal de ladite tige. La tige 10 comporte un index fixe 18 qui peut être par exemple matérialisé par l'aileron 15 ou tout autre moyen.

Le profil extérieur de la calotte 2 présente des graduations ou repères 20 qui correspondent à des empreintes 30 ménagés sur la surface de la sphère 3 afin de parfaitement positionner cette dernière par rapport à la tige 1 en fonction des mesures réalisées sur une pièce d'essai.

Entre l'aileron 15 et la face inclinée 17, la partie métaphysaire 10 est percé d'un trou taraudé 19 qui débouche à l'intérieur de la cavité 11 pour permettre à l'aide d'une vis 4 le blocage de la sphère 3 dans une position déterminée grâce à l'index 18, et aux repères 20.

La vis de blocage 4 coopérant avec le trou 19 pénètre dans la sphère 3 suivant un axe non concourant avec le centre de ladite sphère ce qui empêche tout mouvement relatif de la sphère 3 par rapport à la tige 1.

On constate que l'axe du trou 19 est légèrement incliné par rapport à l'axe vertical de la tige 1 pour permettre une accessibilité constante de la vis 4 lorsque la prothèse est introduite dans le canal huméral.

On note que la combinaison index 18, avec un repère 20 et une empreinte 30 en face de l'axe du trou 19 reproduit une position angulaire combinée des angles α et β préalablement mesurée par exemple sur la pièce d'essai.

La sphère 3 est prévue pleine et directement reliée à la calotte 2 au moyen d'un axe 21 dont la longueur peut varier suivant le cas pathologique. Cette structure oblige d'avoir en stock un certain nombre de sphère 3 solidaire de sa calotte 2 pour pouvoir répondre à toutes les solutions opératoires.

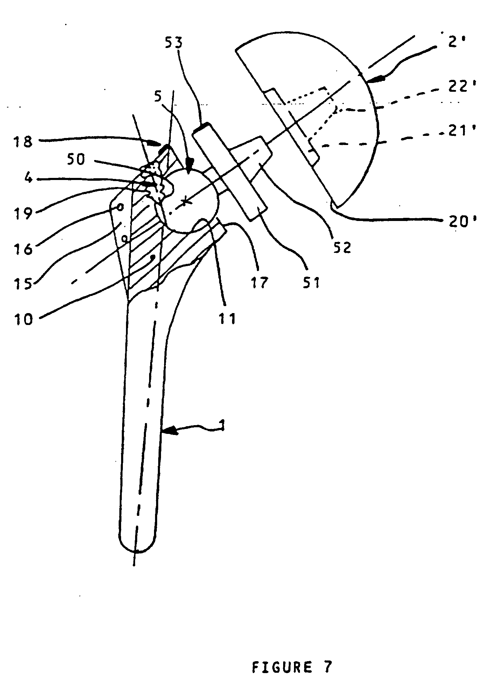

Une première variante représentée en figure 7 consiste en ce que la cavité 11 de la tige 1 reçoit une sphère 5 qui est solidaire d'une collerette circulaire 51 au centre de laquelle s'élève un pion tronconique 52. Ce dernier coopère avec une calotte hémisphériques 2' semblables à celles décrites dans la demande de brevet N° 2 685 633 appartenant au demandeur.

En effet la calotte 2' présente une base 20' dans laquelle est ménagé de manière excentrée un emboîtement circulaire 21' dont le diamètre correspond au jeu près à celui de collerette 51.

Au centre de l'emboîtement 21' est ménagé un alésage tronconique 22' propre à recevoir le pion 52. Le montage et l'immobilisation de la calotte 2' est identique à celui décrit dans la demande de brevet N° 2 685 633.

La vis 4 disposée dans le trou 19 coopère avec des empreintes 50 prévus sur la surface de la sphère 5 permet son blocage dans une position déterminée.

On note que la tige 1 comporte l'index 18 comme cela à été décrit précédemment, tandis que la collerette 51 comporte des repères 53.

La combinaison index 18, avec un repère 53 et une empreinte 50 en face de l'axe du trou 19 reproduit une position angulaire combinée des angles α et β préalablement mesurée sur la pièce d'essai.

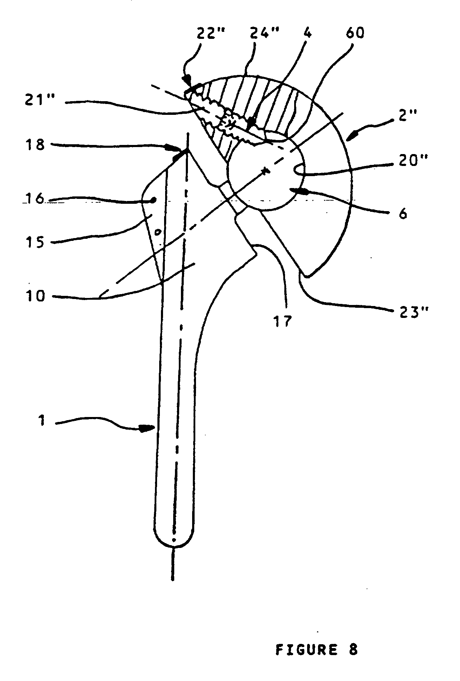

Une seconde variante montrée en figure 8 consiste en ce que la tige 1 est solidaire dans sa partie métaphysaire 10 d'une sphère 6 qui s'étend perpendiculairement à la face inclinée 17. La sphère 6 coopère avec une calotte 2" qui présente un logement 20" en portion de sphère. Un trou taraudé 21" traverse la calotte 2" pour venir déboucher dans le logement 20" afin qu'une vis de pression 4, identique à celle décrite en figure 1, puisse bloquer la calotte sur la sphère 6.

L'axe de trou 21" est prévu non concourant avec le centre du logement 20" et/ou avec la sphère 6 pour permettre une parfaite immobilisation de cette dernière dans ledit logement.

La position de la calotte 2" par rapport à la tige 1 est déterminée au moyen des repères 22" prévus soit sur la base 23", soit sur le profil extérieur 24" de ladite calotte, de l'index fixe 18 prévu sur la tige 1 et des empreintes 60 ménagées sur la surface de la sphère 6.

On note que la combinaison index 18, avec un repère 22" et une empreinte 60 en face de l'axe du trou 21" reproduit une position angulaire combinée des angles α et β préalablement mesurée sur la pièce d'essai.

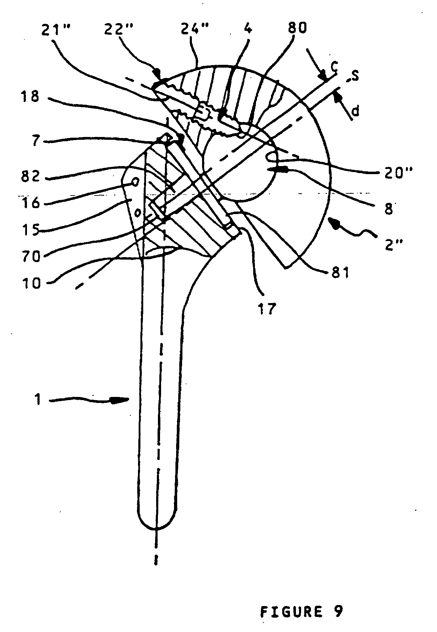

Une troisième variante illustrée en figure 9 consiste en ce que la partie métaphysaire 10 de la tige 1 comporte sur sa face inclinée 17 un logement 7 au milieu duquel est percé un trou non débouchant 70 à profil tronconique. Le logement 7 est prévu pour recevoir une collerette 81 solidaire d'un pion tronconique 82 qui coopère avec le trou 70 pour le blocage par coincement de ladite collerette 81 sur la tige 1. La collerette 81 présente à l'opposé du pion tronconique 82 une sphère 8 qui coopère avec le logement 20" de la calotte 2" décrite précédemment en figure 8.

On constate que l'axe S de la sphère 8 est décalé d'une distance d par rapport à l'axe C de la collerette 81 emboîté sans jeu dans le logement 7 de la tige 1 pour permettre une excentration de la calotte 2".

La position de la calotte 2" par rapport à la tige 1 est déterminée au moyen des repères 22" prévus soit sur la base 23", soit sur le profil extérieur 24" de ladite calotte, de l'index fixe 18 prévu soit sur la tige 1 soit sur la collerette 81 et des empreintes 80 ménagées sur la surface de la sphère 8.

On note que la combinaison index 18, avec un repère 22" et une empreinte 80 en face de l'axe du trou 21" reproduit une position angulaire combinée des angles α et β préalablement mesurée sur la pièce d'essai.

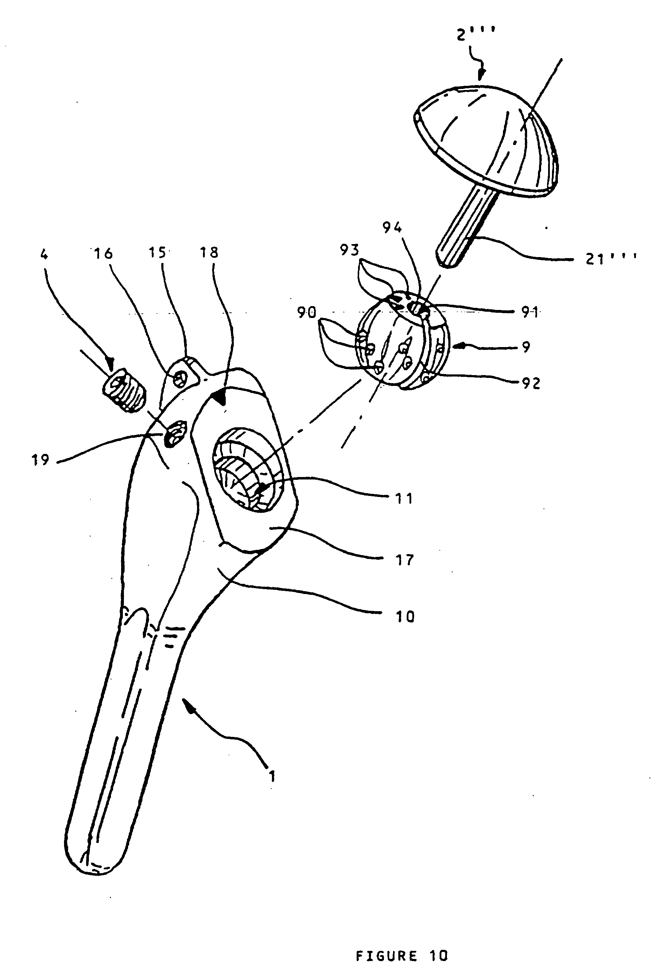

Une quatrième variante suivant l'invention représentée en figure 10 consiste en ce que la tige 1 est identique à celle décrite précédemment en figure 1 afin que sa cavité 11 coopère avec une sphère 9 semblable à celle décrite dans la demande de brevet FR 2 727 002 appartenant au demandeur.

La sphère 9 comprend en son milieu un alésage 91 dans lequel débouche une fente radiale 92. L'alésage 91 est prévu pour recevoir une tige 21''' solidaire et décalée latéralement d'une distance d par rapport au milieu de la calotte 2"' pour offrir une excentration de cette dernière.

Le fait que la calotte 2"' soit indépendante de la sphère 9 permet un réglage en hauteur de cette dernière par rapport à la partie métaphysaire 10 de la tige 1.

On constate dans cette réalisation que la vis 4 coopérant avec le trou 19 permet le blocage d'une part de la sphère 9 dans la cavité 11 et d'autre part la fixation de la tige 21"' dans l'alésage 91.

La fixation peut être complété par une autre vis de serrage pour améliorer la fixation de la tige 21"' dans l'alésage 91.

La position de la calotte 2"' par rapport à la tige 1 est déterminée au moyen des repères 93 prévus sur les pôles tronqués 94 de la sphère 9, de l'index fixe 18 prévu sur la tige 1 et des empreintes 90 ménagées sur la surface de la sphère 9.

On note que la combinaison index 18, avec un repère 93 et une empreinte 90 en face de l'axe du trou 19 reproduit une position angulaire combinée des angles α et β préalablement mesurée sur la pièce d'essai.

Le réglage des prothèses humérale décrites ci-dessus est obtenus au moyen d'une pièce d'essai ou fantôme identique à la prothèse à fixer. Cette pièce d'essai permet ainsi au chirurgien de mesurer parfaitement la position relative calotte/sphère par rapport à la tige 1 en fonction de deux angles α et β. A chaque association de ses deux angles correspond un repère 20, 53, 22", 93 à utiliser sur les prothèses à implanter. Les angles α et β sont définis par rapport à l'axe de référence qui est l'axe de la tige ou du canal huméral.

Ainsi l'angle α est l'angle d'inclinaison dans le plan frontal, tandis que l'angle β est l'angle d'ante/ou rétroversion par rapport au plan frontal autour de l'axe de la tige. L'angle β correspond à l'angle de rotation de la tête humérale, constituée par la sphère 3, 5, 6, 8, 9 et/ou la calotte 2, 2', 2", 2"', autour de l'axe de la tige 1 par rapport au plan frontal.

Le chirurgien reporte les mesures aux tolérances près relevés sur la pièce d'essai sur la prothèse huméral à implanter grâce à l'index fixe 18, aux graduations ou repères 20, 53, 22", 93 et aux empreintes 30, 50, 60, 80, 90 pour que la sphère 3, 5, 6, 8, 9 et/ou la calotte 2, 2', 2", 2"' se trouve par rapport à la tige 1 positionnée suivant la combinaison des angles α et β.

Le chirurgien bloque la sphère 3, 5, 6, 8, 9 et/ou la calotte 2, 2', 2", 2''' dans la position déterminée au moyen de la vis de serrage 4 avant d'implanter la prothèse dans le canal huméral du patient.

## Revendications

1. Prothèse humérale à sphère comprenant une tige (1) pouvant s'ancrer dans le canal huméral, une calotte hémisphérique (2, 2', 2", 2"') propre à coopérer avec la glène de l'épaule et une sphère (3, 5, 6, 8, 9) réalisant la liaison entre la tige et la calotte, **caractérisée en ce qu'**elle comprend des moyens d'indexation de la position de la sphère (3, 5, 6, 8, 9) et/ou de la calotte (2, 2', 2", 2"') par rapport à la tige (1) qui sont constitués par la combinaison :
• d'un index fixe (18) situé sur la tige (1) avec un repère (20, 53, 22",93) situé sur la sphère (3, 5, 6, 8, 9) et/ou la calotte (2, 2', 2", 2"') ;
• et d'une empreinte (30, 50, 60, 80, 90) ménagée dans la sphère (3, 5, 6, 8, 9) avec un trou (19, 21") percé dans la partie métaphysaire (20) de la tige (1) ou dans la calotte (2"),
les dits moyens d'indexation permettant de positionner la sphère (3, 5, 6, 8, 9) et/ou la calotte (2, 2', 2", 2''') dans un nombre fini de positions angulaires combinées α et β de la calotte par rapport à la tige, qui sont obtenues des mesures relevées sur une pièce d'essai ou tout autre moyen, afin de bloquer par des moyens de serrage (4) ladite sphère et/ou ladite calotte sur la tige (1) avant sa mise en place dans le canal huméral, lesdits angles α et β représentant respectivement l'angle d'inclinaison dans le plan frontal, et l'angle d'ante ou rétroversion par rapport au plan frontal autour de l'axe de la tige.

2. Prothèse humérale suivant le revendication 1, **caractérisée en ce que** le trou (19, 21") recevant les moyens de serrage (4) est disposé suivant un axe non concourant avec le centre de la sphère (3, 5, 6, 8, 9) et/ou la calotte (2, 2', 2", 2"') pour empêcher tout mouvement relatif entre la sphère et/ou la calotte par rapport à la tige (1).

3. Prothèse humérale suivant la revendication 1, **caractérisée en ce que** la tige (1) comporte dans sa partie métaphysaire (10) une cavité (11 ) propre à recevoir une sphère (3, 5, 9) coopérant avec la calotte (2, 2', 2"'), tandis que des moyens de serrage (4) prévus sur la tige permettent l'immobilisation de la sphère et de la calotte.

4. Prothèse humérale suivant la revendication 1, **caractérisée en ce que** la tige (1) est solidaire dans sa partie métaphysaire (10) d'une sphère (6, 8) qui coopère avec un logement (20") ménagé dans la calotte (2"), tandis que des moyens de serrage (4) prévus sur la calotte permettent son immobilisation sur la sphère.

5. Prothèse humérale suivant la revendication 4, **caractérisée en ce que** la tige (1) comporte dans sa partie métaphysaire (10) un logement (7) au milieu duquel est percé un trou (70) à profil tronçonique recevant un pion (82) de même profil s'étendant d'une collerette (81) disposée dans le logement (7), ladite collerette étant solidaire à l'opposé du pion (82) d'une sphère (8) qui coopère avec le logement (20") prévu dans la calotte (2").

6. Prothèse humérale suivant la revendication 5, **caractérisée en ce que** l'axe (S) de la sphère (8) est décalé latéralement d'une distance (d) par rapport à l'axe (C) de la collerette (81) pour permettre une excentration de la calotte (2").

## Patentansprüche

1. Oberarmknochenprothese mit Kugel, die einen Schaft (1) umfasst, der sich im Oberarmknochenkanal verankern kann, einer halbkugelförmigen Kalotte (2, 2', 2", 2"'), die mit der Gelenkpfanne der Schulter zusammenarbeiten kann, und einer Kugel (3, 5, 6, 8, 9), die die Verbindung zwischen dem Schaft und der Kalotte herstellt, **dadurch gekennzeichnet, dass** sie Mittel zum Positionieren der Position der Kugel (3, 5, 6, 8, 9) und/oder der Kalotte (2, 2', 2", 2"') zum Schaft (1) umfasst, die aus der folgenden Kombination bestehen:
• eine stationäre Markierung (18), die auf dem Schaft (1) liegt, mit einer Kennzeichnung (20, 53, 22", 93), die auf der Kugel (3, 5, 6, 8, 9) und/oder der Kalotte (2, 2', 2", 2"') liegt;
• ein Abdruck (30, 50, 60, 80, 90), der in der Kugel (3, 5, 6, 8, 9) mit einer Bohrung (19, 21") eingerichtet ist, die in den metaphysären Teil (20) des Schafts (1) oder in die Kalotte (2") gebohrt ist,
wobei es die Positionierungsmittel erlauben, die Kugel (3, 5, 6, 8, 9) und/oder die Kalotte (2, 2', 2", 2"') in einer finiten Anzahl kombinierter Winkelstellungen α und β der Kalotte zum Schaft zu positionieren, die aus den Messungen erzielt werden, die auf einem Testteil oder jedem anderen Mittel abgenommen werden, um durch Spannmittel (4) die Kugel und/oder die Kalotte auf dem Schaft (1) vor seinem Anbringen im Oberarmknochenkanal zu blockieren, wobei die Winkel α und β jeweils den Neigungswinkel in der frontalen Ebene und den Winkel des Vorwärts- oder Rückwärtsbeugens zur frontalen Ebene um die Achse des Schafts darstellen.

2. Oberarmknochenprothese nach Anspruch 1, **dadurch gekennzeichnet, dass** die Bohrung (19, 21"), die die Spannmittel (4) aufnimmt, entlang einer Achse angeordnet ist, die nicht mit der Mitte der Kugel (3, 5, 6, 8, 9) und/oder der Kalotte (2, 2', 2", 2"') zusammenfällt, um jede relative Bewegung zwischen der Kugel und/oder der Kalotte zum Schaft (1) zu verhindern.

3. Oberarmknochenprothese nach Anspruch 1, **dadurch gekennzeichnet, dass** der Schaft (1) in seinem metaphysären Teil (10) einen Hohlraum (11) umfasst, der eine Kugel (3, 5, 9) aufnehmen kann, die mit der Kalotte (2, 2', 2"') zusammenwirkt, während Spannmittel (4), die auf dem Schaft vorgesehen sind, das Stillstellen der Kugel und der Kalotte erlauben.

4. Oberarmknochenprothese nach Anspruch 1, **dadurch gekennzeichnet, dass** der Schaft (1) in seinem metaphysären Teil (10) fest mit einer Kugel (6, 8) verbunden ist, die mit einer Aufnahme (20") zusammenwirkt, die in der Kalotte (2") eingerichtet ist, während Spannmittel (4), die auf der Kalotte vorgesehen sind, sein Stillstellen auf der Kugel erlauben.

5. Oberarmknochenprothese nach Anspruch 4, **dadurch gekennzeichnet, dass** der Schaft (1) in seinem metaphysären Teil (10) eine Aufnahme (7) umfasst, in deren Mitte eine Bohrung (70) mit kegelstumpfförmigem Profil gebohrt ist, die einen Zapfen (82) mit gleichem Profil aufnimmt, der sich von einem Kragen (81), der in der Aufnahme (7) angeordnet ist, erstreckt, wobei der Kragen gegenüber dem Zapfen (82) fest mit einer Kugel (8) verbunden ist, die mit der Aufnahme (20"), die in der Kalotte (2") vorgesehen ist, zusammenwirkt.

6. Oberarmknochenprothese nach Anspruch 5, **dadurch gekennzeichnet, dass** die Achse (S) der Kugel (8) seitlich um eine Entfernung (d) zur Achse (C) des Kragens (81) versetzt ist, um ein Exzentrieren der Kalotte (2") zu erlauben.

## Claims

1. Humeral prosthesis with a sphere, comprising a rod (1) which can be anchored into the humeral canal, a hemispherical cup (2, 2', 2", 2"') which can interact with the shoulder socket and a sphere (3, 5, 6, 8, 9) providing the connection between the rod and the cup, **characterized in that** it comprises means for indexing the position of the sphere (3, 5, 6, 8, 9) and/or the cup (2, 2', 2", 2"') with respect to the rod (1), which means consist of the combination:
• of a fixed pointer (18) located on the rod (1) with a reference mark (20, 53, 22", 93) located on the sphere (3, 5, 6, 8, 9) and/or the cup (2, 2', 2", 2"');
• and of a recess (30, 50, 60, 80, 90) formed in the sphere (3, 5, 6, 8, 9) with a hole (19, 21") pierced in the metaphyseal part (20) of the rod (1) or in the cup (2"),
the said indexing means allowing the sphere (3, 5, 6, 8, 9) and/or the cup (2, 2', 2", 2"') to be positioned in a finite number of combined angular positions α and β of the cup with respect to the rod, which positions are obtained from the measurements read off from a test piece or some other means in order, using binding means (4), to immobilize the said sphere and/or the said cup on the rod (1) before it is fitted in the humeral canal, the said angles α and β respectively representing the angle of inclination in the frontal plane, and the anteversion or retroversion angle with respect to the frontal plane about the axis of the rod.

2. Humeral prosthesis according to Claim 1, **characterized in that** the hole (19, 21") that receives the binding means (4) is located along an axis that does not run through the centre of the sphere (3, 5, 6, 8, 9) and/or the cup (2, 2', 2", 2"') so as to prevent any relative movement between the sphere and/or the cup with respect to the rod (1).

3. Humeral prosthesis according to Claim 1, **characterized in that** the rod (1) in its metaphyseal region (10) comprises a cavity (11) capable of accommodating a sphere (3, 5, 9) interacting with the cup (2, 2', 2"'), while binding means (4) provided on the rod allow the sphere and the cup to be immobilized.

4. Humeral prosthesis according to Claim 1, **characterized in that** the rod (1) in its metaphyseal region (10) is secured to a sphere (6, 8) which interacts with a housing (20") formed in the cup (2"), while binding means (4) provided on the cup allow it to be immobilized on the sphere.

5. Humeral prosthesis according to Claim 4, **characterized in that** the rod (1) in its metaphyseal region (10) comprises a housing (7) in the middle of which there is drilled a hole (70) with a tapering profile receiving a peg (82) of the same profile extending from a flange (81) placed in the housing (7), the said flange being secured, on the opposite side from the peg (82), to a sphere (8) which interacts with the housing (20") provided in the cup (2").

6. Humeral prosthesis according to Claim 5, **characterized in that** the axis (S) of the sphere (8) is laterally offset by a distance (d) with respect to the axis (C) of the flange (81), so as to allow the cup (2") to be offcentred.
